# EUROPEAN PATENT APPLICATION

(11) **EP 4 438 739 A1**
(43) Date of publication of application: **02.10.2024**
(21) Application number: 22897156.0
(22) Date of filing: 04.07.2022
(51) Int. Cl.: C12Q 1/6844

(54) **REVERSE-TRANSCRIPTION AMPLIFICATION SYSTEM AND METHOD BASED ON RECOMBINASE POLYMERASE AMPLIFICATION TECHNOLOGY**

(30) Priority: 23.11.2021 CN 202111392581
(71) Applicant: Daan Gene Co., Ltd., Guangzhou, Guangdong 510665 (CN)
(72) Inventor: JIANG, Xiwen, Guangzhou, Guangdong 510665 (CN); LIU, Aishan, Guangzhou, Guangdong 510665 (CN); WANG, Zhouquan, Guangzhou, Guangdong 510665 (CN); ZHU, Wenya, Guangzhou, Guangdong 510665 (CN)
(74) Representative: Diehl & Partner
(86) International application number: PCT/CN2022/103696
(87) International publication number: WO 2023/093060

(57) **Abstract**

Provided are a reverse-transcription amplification system and method based on recombinase polymerase amplification (RPA) technology, specifically, provided are that an RNA template in a sample can be effectively amplified after a reverse transcriptase and RNasin are added to an RPA system, and on this basis, the nucleic acid amplification efficiency of a reverse-transcription real-time fluorescent RPA system (RT-qRPA) can be effectively improved by means of adding inorganic pyrophosphatase (IPP).

## Description

### TECHNICAL FIELD

The invention belongs to the technical field of biology, and particularly relates to a reverse-transcription amplification system and method based on recombinase polymerase amplification technology.

### TECHNICAL BACKGROUND

The nucleic acid detection method with high sensitivity and high selectivity has always been an important scientific experimental tool for molecular biology and medical research. At present, the commonly used quantitative real-time polymerase chain reaction (qPCR) has been widely used in the field of nucleic acid detection due to its high accuracy, repeatability and fast detection speed. However, qPCR needs to repeatedly change and maintain the reaction system at different temperatures during the reaction process, resulting in that a qPCR instrument capable of precisely controlling the temperature is generally required for qPCR detection to allow the reaction, limiting the application site of qPCR.

The isothermal amplification technology is a technology for carrying out reaction at a constant temperature in the nucleic acid amplification process. As a qPCR instrument which is necessary for the qPCR technology is not needed, the cost of the instrument can be greatly saved, and the heating instrument which does not need precise temperature control can be made into a small size for carrying, so that the application of the isothermal amplification technology is greatly facilitated. Recombinase polymerase amplification (RPA) is one of the isothermal amplification techniques, and it has attracted more and more attention in the field of nucleic acid detection due to its high efficiency. However, when RPA technology is used to amplify RNA, there are problems of low amplification efficiency and poor specificity. Therefore, those skilled in the art are committed to developing a reverse-transcription amplification system and method based on recombinase polymerase amplification technology with high sensitivity, strong specificity, and accurate detection results.

### SUMMARY OF THE INVENTION

The invention aims to provide a reverse-transcription amplification system and method based on recombinase polymerase amplification technology with high sensitivity, strong specificity and accurate detection result.

In a first aspect of the present invention, there is provided a reverse-transcription amplification reagent combination based on recombinase polymerase amplification technology, the reagent combination comprising a recombinase, a single-stranded binding protein, a DNA polymerase, a reverse transcriptase, and a ribonuclease inhibitor protein.

In another preferred embodiment, said reagent combination further comprises an inorganic pyrophosphatase.

In another preferred embodiment, said reagent combination further comprises a loading protein; preferably, the loading protein is loading protein UvsY.

In another preferred embodiment, said reagent combination further comprises an exonuclease; preferably, the exonuclease is Exonuclease III (EXO).

In another preferred embodiment, said reagent combination further comprises Creatine Kinase (CK).

In another preferred embodiment, said recombinase is recombinase UvsX.

In another preferred embodiment, said single-chain binding protein is single-chain binding protein gp32.

In another preferred embodiment, said DNA polymerase is DNA polymerase Bsu.

In another preferred embodiment, said reverse transcriptase is reverse transcriptase MMLV.

In another preferred embodiment, said reagent combination further comprises one or more components selected from the group consisting of:
Creatine phosphate, Tris-HCl, ATP, DTT, dNTPs, potassium acetate, magnesium acetate, and polyethylene glycol (preferably PEG35000).

In a second aspect of the invention, there is provided a kit comprising the reagent combination of the first aspect of the invention.

In another preferred embodiment, said kit further comprises specific primers and/or probes.

In another preferred embodiment, said kit comprises a first container having one or more protein components selected from the group consisting of a recombinase, single-stranded binding protein, DNA polymerase, reverse transcriptase, ribonuclease inhibitor protein, inorganic pyrophosphatase, loading protein, nucleic acid exonuclease, creatine kinase.

In another preferred embodiment, each of said protein components is a lyophilized powder.

In another preferred embodiment, said kit further comprises a second container having one or more components selected from the group consisting of creatine phosphate, Tris-HCl, ATP, DTT, dNTPs, potassium acetate, and polyethylene glycol.

In another preferred embodiment, said kit further comprises a third container having magnesium acetate.

In another preferred embodiment, said kit further comprises a fourth container having water or a buffer.

In a third aspect of the present invention, there is provided a method for reverse-transcription amplification of RNA in a sample based on recombinase polymerase amplification technology, the method comprising the steps of:
(1) preparation of an RT-RPA amplification system
   The RT-RPA amplification system comprises a reagent combination according to the first aspect of the invention, an RNA template, and primers that specifically amplify a reverse-transcription product of the RNA template;
(2) amplification reaction

The amplification reaction is carried out under an isothermal condition.

In another preferred embodiment, the RT-RPA amplification system further comprises a probe that specifically targets the reverse-transcription product of the RNA template.

In another preferred embodiment, that probe has a fluorescent label.

It is to be understood that within that scope of the present invention, each of the above-described technical features of the present invention and each of the technical features specifically described hereinafter (as embodiments) can be combined with each other to constitute new or preferred technical solutions. Due to limited space, they will not be repeated herein.

### BRIEF DESCRIPTION OF THE DRAWINGS

Fig. 1 shows the detection results in Example 1.
Fig. 2 shows the detection results of the reaction system with and without the addition of IPP enzyme.

### DETAILED DESCRIPTION OF THE INVENTION

Through extensive and in-depth research, the inventor unexpectedly found that after the reverse transcriptase (MMLV) and ribonuclease inhibitor protein (RNasin) are added into the RPA system, the RNA template in the sample can be effectively amplified, and on the basis, the addition of the inorganic pyrophosphatase (IPP) can effectively improve the nucleic acid amplification efficiency of the reverse transcription real-time fluorescent RPA system (RT-qRPA).

Before describing the invention, it is to be understood that the invention is not limited to the specific methods and experimental conditions described, as such methods and conditions may be varied. It should also be understood that the terminology used herein is for the purpose of describing particular embodiments only and is not intended to be limiting, the scope of the invention being limited only by the appended claims.

Unless otherwise defined, all technical and scientific terms used herein have the same meaning as commonly understood by one of ordinary skill in the art to which this invention belongs. As used herein, the term "about" when used in reference to a specifically recited value means that the value may vary from the recited value by no more than 1%. For example, as used herein, the expression "about 100" includes all values between 99 and 101 (e.g., 99.1, 99.2, 99.3, 99.4, etc.).

While any method and material similar or equivalent to those described in the present invention may be used in the practice or testing of the invention, preferred methods and materials are exemplified herein.

### Recombinase polymerase amplification technology

Recombinase polymerase amplification (RPA) is one of the isothermal amplification techniques. Disclosure of RPA methods, including US patent documents US 7, 270, 981; US 7,399,590; US 7,666,598; US 7,435,561; US patent publication US 2009/0029421; International application WO 2010/141940.

The key enzymes of RPA system include a recombinase that can bind single-stranded nucleic acid (oligonucleotide primer), a single-stranded DNA binding protein (SSB) and a strand displacement DNA polymerase. The protein-DNA complex formed by the combination of recombinase and primer can search for homologous sequence in double-stranded DNA. Once the primers locate the homologous sequence, a strand exchange reaction occurs to form and initiate DNA synthesis, and the target region on the template is exponentially amplified. The replaced DNA strand binds to SSB to prevent further replacement.

The recombinase can pair oligonucleotide primers homologous to the double-stranded DNA template when the RPA reaction starts. When a nucleotide template is present, an exponential amplification reaction is initiated with two or more sequence-specific primers (e.g., gene-specific). The reaction progresses rapidly, and specific amplification of the double-stranded DNA template sequence results in amplification of the DNA template from only a few copies to a detectable level within minutes.

A reverse transcriptase, ribonuclease inhibitor protein and inorganic pyrophosphatase are added on the basis of a real-time fluorescent RPA technology in the invention, the reverse transcriptase and the ribonuclease inhibitor protein are simultaneously used in a reaction system to start and maintain normal progress of an RNA template amplification reaction, and the reaction efficiency is remarkably improved after adding the inorganic pyrophosphatase.

### Recombinase

The recombinase (recombinant protein) for the RPA reaction may be derived from prokaryotes, viruses, or eukaryotes. Typical recombinases include UvsX and RecA (e.g., RecA protein or UvsX protein obtained from any species), and fragments or mutants thereof, or any combination thereof.

The RecA and UvsX proteins may be obtained from any species and the RecA and UvsX fragments or mutant proteins may also be produced using suitable RecA and UvsX proteins, nucleotide sequences and molecular biology techniques (see formation of UvsX mutants as described in US 8,071,308). Typical UvsX proteins include those derived from myoviridae phages proteins such as T4, T2, T6, Rb69, Aehl, KVP40, Acinetobacter phage 133, Aeromonas phage 65, Cyanophage P-SSM2, cyanophage PSSM4, cyanophage S-PM2, RB14, Rb32, Aeromonas phage 25, vibrio bacteriophage nt-1, phi-1, Rb16, Rb43, bacteriophage 31, bacteriophage 44RR2.8t, Rb49, bacteriophage RB3, and bacteriophage LZ2. Other typical recombinase proteins include archaeal RADA and RADB proteins, and eukaryotic (e.g., plant, mammalian, and fungal) Rad51 proteins (e.g., Rad51, Rad51B, Rad51C, Rad51D, DMC 1, XRCC 2, XRCC 3, and recA) (see e.g. Lin et al., proc. Natl. Acad. Sc. U.S.A. 103: 10328-10333, 2006).

In a preferred embodiment of the present invention, the recombinase is recombinase UvsX, and a typical UvsX has an amino acid sequence shown in NCBI Seq No. NP_049656.2.

### Single-chain binding protein

Single-chain binding proteins (single-chain DNA binding proteins) can be used to stabilize nucleotides and one or more single-chain DNA binding proteins can be derived from or obtained from a variety of species, such as prokaryotes, viruses, or eukaryotes. Typical single-stranded DNA binding proteins include, but are not limited to, Escherichia coli SSB and those derived from myoviral bacteriophage, such as T4, T2, T6, Rb69, Aehl, KVP40, Acinetobacter bacteriophage 133, Aeromonas bacteriophage 65, cyanophage, vibrio bacteriophage, and the like.

In the present invention, preferably the single-chain binding protein is the single-chain binding protein gp32. gp32 protein plays a key role in the DNA replication, recombination, and repair of T4 bacteriophage. The most important thing is that gp32 protein has the property of tightly binding to single-stranded DNA. For the amino acid sequence of typical gp32 protein, please refer to NCBI Seq No. NP_049854.

### DNA polymerase

The DNA polymerase may be an eukaryotic or prokaryotic polymerase. Examples of eukaryotic polymerases include pol-alpha, pol-beta, pol-delta, pol-epsilon, and mutants or fragments thereof, or combinations thereof. Examples of prokaryotic polymerases include *E.coli* DNA polymerase i (e.g., Klenow fragment), bacteriophage T4 gp43 DNA polymerase, a large fragment of Bacillus Stearothermophilus Polymerase I, Phi-29 DNA polymerase, T7 DNA polymerase, Bacillus subtilis Pol I, Staphylococcus aureus PolI, Escherichia coli DNA polymerase I, Escherichia coli DNA polymerase II, Escherichia coli DNA polymerase III, Escherichia coli DNA polymerase IV, Escherichia coli DNA polymerase V, and mutants or fragments thereof, or combinations thereof.

In the present invention, preferably the DNA polymerase is Bsu DNA polymerase. In general, the Bsu DNA polymerase (Bsu DNA polymerase, Large fragment) is derived from Bacillus subtilis by truncating the first 296 codons of the Bacillus subtilis DNA polymerase I gene. This fragment retains 5'-3' polymerase activity of Bsu DNA polymerase but lacks 5'-3' exonuclease activity while that large fragment itself lacks 3'-5' exonuclease activity. The amino acid sequence of a typical Bsu DNA polymerase is shown in NCBI Seq No. AAR00931.1.

### Loading protein

The loading protein may be derived from prokaryote, viruses, or eukaryotes. Typical loading proteins include E. coli RecO, E. coli RecR, UvsY, and mutants or fragments thereof, or combinations thereof. Typical UvsY proteins include those derived from myoviral bacteriophages such as T4, T2, T6, Rb69, Aehl, KVP40, acinetobacter bacteriophage 133, aeromonas bacteriophage 65, cyanophage P-SSM2, cyanophage PSSM4, cyanophage S-PM2, Rb14, Rb32, aeromonas bacteriophage 25, vibrio bacteriophage nt-1, phi-1, Rb16, Rb43, bacteriophage 31, bacteriophage 44RR2.8t, Rb49, bacteriophage Rb3, and bacteriophage LZ2.

In the present invention, preferably the loading protein is the loading protein UvsY. The amino acid sequence of a typical loading protein, UvsY, is shown in NCBI Seq No. NP_049799.2.

### Exonuclease

Exonuclease is a general term for several subclasses of hydrolases (EC3.1.11.13.14.15.16), which is able to catalyze the hydrolysis of phosphodiester bonds at the ends of nucleic acid chains and cleave nucleotides one by one. According to the substrate specificity, it is divided into DNA exonuclease, RNA exonuclease and nucleic acid exonuclease that can act on both DNA and RNA.

In a preferred embodiment of the invention, the exonuclease of the invention is the Exonuclease III. The amino acid sequence of a typical Exonuclease III is shown in NCBI Seq No. WP_000673937.1.

### Creatine kinase

Creatine Kinase (CK) is an important kinase that is directly related to energy transfer in cells, muscle contraction, and ATP regeneration. It reversibly catalyzes the transphosphorylation reaction between creatine and ATP.

The creatine kinase may be selected from rabbit muscle kinase, carp muscle kinase, such as carp muscle kinase M1 type (M1-CK). The amino acid sequence of a typical creatine kinase is shown in NCBI Seq No. NP_001075708.1.

### Reverse transcriptase

Reverse transcriptase is also known as RNA-dependent DNA polymerase. The enzyme uses RNA as a template, dNTP as a substrate, and tRNA (mainly tryptophan tRNA) as a primer to synthesize a DNA single strand complementary to the RNA template in the 5'-3' direction on the 3'-OH end of tRNA according to the principle of base pairing, which is called complementary DNA (cDNA).

Reverse transcriptase can be used for synthesis of first strand cDNA, preparation of cDNA probes, RNA transcription, sequencing and reverse transcription of RNA. RT commonly used in the art include murine leukemia virus (M-MLV) RT and avian myeloblastoma virus (AMV) RT. The amino acid sequence of a typical creatine kinase is shown in NCBI Seq No. AAA66622.1.

### Ribonuclease inhibitor protein

Ribonuclease inhibitor protein (RNasin) is a protein inhibitor of RNase (RNasin). It can be extracted from rat liver or human blastocyst, or prepared by gene recombination. Rnasin is a noncompetitive inhibitor of RNase that binds to and inactivates a variety of RNases. The amino acid sequence of a typical RNasin is shown in NCBI Seq No. P13489.2.

### Inorganic pyrophosphatase

Inorganic pyrophosphatase (IPP) catalyzes the hydrolysis of inorganic pyrophosphate to orthophosphate. Inorganic pyrophosphatase can be prepared by recombinant *E. coli* strains or yeast strains carrying the inorganic pyrophosphatase gene. The amino acid sequence of a typical inorganic pyrophosphatase is shown in NCBI Seq No. WP_000055072.1.

### RT-RPA reaction system and detection method

In a preferred embodiment, the RT-RPA reaction system of the present invention comprises:
Recombinant protein UvsX, loading protein UvsY, single-chain binding protein gp32, Exonuclease III, Bsu DNA polymerase, creatine kinase CK, reverse transcriptase (MMLV), RNasin, IPP enzyme, creatine phosphate (PC), Tris-HCl(pH7.5), ATP, dithiothreitol (DTT), dNTPs, potassium acetate (KOAc), magnesium acetate, polyethylene glycol (e.g.PEG35000).

In another preferred embodiment, that contents of the components in the RT-RPA reaction system are as follows:

| Component | Content |
|---|---|
| Recombinant protein UvsX | 100-1000ng/µL |
| Loading protein UvsY | 100-500ngµL |
| Single-chain binding protein gp32 | 500-2000ng/µL |
| Exonuclease III | 1-10ng/µL |
| Bsu DNA polymerase | 5-15ng/µL |
| Creatine kinase CK | 0.1-5ng/µL |
| Reverse transcriptase (MMLV) | 1-10U/µL |
| RNasin | 1-50U/µL |
| IPP enzyme | 0.1-5U/µL |
| Creatine phosphate (PC) | 50-500mM |
| Tris-HCl (pH7.5) | 5-20mM |
| ATP | 5-20mM |
| Dithiothreitol (DTT) | 0.1-5mM |
| dNTPs | 50-500µM |
| Potassium acetate (KOAc) | 10-100mM |
| Magnesium acetate | 5-25 mM |
| PEG35000 | 0.5-10% (w/w) |

Preferably, the concentration of upstream primer in the RT-RPA reaction system is 10-200nM, that of downstream primer is 100nM, that of probe is 50-300nM, and that of RNA template is 1-10 µL. Preferably, that total volume of the RT-qRPA system is 15 to 50 µL, with the remainder been supplemented with double distilled water.

Reaction procedures:
The temperature is maintained at 20-45 °C and the reaction continues for 10-40 minutes.

Reaction endpoints are monitored by agarose gel electrophoresis, Sybr green I, or specific probes. When monitored by Sybr green I, in the reaction system, SYBR Green I is increased to the final concentration of 0.3-0.5x. The reaction time is within 20-40 minutes, and the fluorescence is read once every 30 s. The instruments for detection can be ABI7500, FTC-3000, Bio-Rad CFX MiniOpticon System, GenDx Thermofluorescence Detector GS8, etc.

If the amplification system is detected by using a specific probe, the final concentration of the fluorescence-labeled probe is 10-500nM. The fluorescence detection uses ABI QuantStudio 5, ABI7500, FTC-3000, Bio-Rad CFX MiniOpticon System, GenDx Thermofluorescence Detector GS8, etc.

### The invention has the main advantages as follows:

(1) The present invention establishes a reverse transcription amplification system (RT-RPA system) based on a recombinase polymerase amplification technology.
(2) The reverse-transcription real-time fluorescent RPA system of the invention has extremely high nucleic acid amplification efficiency.
(3) After the IPP enzyme is added to the reverse-transcription real-time fluorescent RPA system, the detection sensitivity can be improved by 10 times.

The invention is further described below in detail in connection with specific examples. It is to be understood that these examples are merely illustrative of the invention and are not intended to limit the scope of the invention. The experimental method without specifying the detailed conditions in the following examples is usually performed under conventional conditions such as those described in SAMBROOK.J. et al. Molecular cloning: a laboratory manual (Huang Peitang *et al.,* Beijing: Science Press, 2002), or conditions recommended by the manufacturer. Percentages and parts are by weight unless otherwise stated. The experimental materials and reagents used in the following examples are available from commercial sources unless otherwise specified.

### Example 1

The RT-RPA reaction system employed in this example was as follows:

| Component | Content |
|---|---|
| Recombinant protein UvsX | 500ng/µL |
| Loading protein UvsY | 228ngµL |
| Single-chain binding protein gp32 | 919ng/µL |
| Exonuclease III | 3ng/µL |
| Bsu DNA polymerase | 9.5ng/µL |
| Creatine kinase CK | 1ng/µL |
| Reverse transcriptase (MMLV) | 5U/µL |
| RNasin | 20U/µL |
| IPP enzyme | 1U/µL |
| Creatine phosphate (PC) | 200mM |
| Tris-HCl (pH7.5) | 10mM |
| ATP | 10mM |
| Dithiothreitol (DTT) | 1mM |
| dNTPs | 200µM |
| Potassium acetate (KOAc) | 50mM |
| Magnesium acetate | 15 mM |
| PEG35000 | 5% |

According to the table, 100nM reaction upstream primer, 100nM reaction downstream primer, 150nM reaction probe and 8µL reaction RNA template with different concentrations were added into the RPA basic reaction solution configuration to form a complete RT-qRPA system. The total system volume was 20µL, and double distilled water were supplemented to 20µL.

Reaction procedures:
The temperature was maintained at 42°C for 20 minutes using the FAM channel and the fluorescence signal was detected every 30 seconds on an ABI QuantStudio 5 quantitative PCR instrument.

The detection target is apoB gene: 
cagtgtatctggaaagcctacaggacaccaaaataaccttaatcatcaattggttacaggaggctttaagttcagc
atctttggctcacatgaaggccaaattccgagagactctagaagatacacgagaccgaatgtatcaaatggacat
tcagcaggaacttcaacgatacctgtctctggtaggccaggtttatagcacacttgtcacctacatttctgattggtggact
cttgctgctaagaaccttactgactttgcagagcaatattctatccaagattgggctaaacgtatgaaagca（SEQ ID
NO.1）
Upstream primer: CAGTGTATCTGGAAAGCCTACGGACACCAAAA (SEQ ID NO.2)
Downstream Primer: TGCTTTCATACGTTTTAGCCCATTGGATG ( SEQ ID NO.3)
Probe:
   attcagcaggaacttcaacgatacctgtctc Tg-THF-Taggccaggtttatagca[C3-spacer] (SEQ ID NO.4)
The 32nd base is T, which is modified into a base with FAM fluorescent group;
A THF (tetrahydrofuran) site was added between base 33 and 34;
The 34th base is T, which is modified into a base with a BHQ1 quenching group;
C3-spacer is added at the 3' end.

The apoB gene shown above was cloned into a plasmid, and a T7 *in vitro* transcription promotor, TAATACGCACTCACTATAGG (SEQ ID NO.5) was added at the 5' end of the template by PCR, transcription was performed through recognition and initiation by T7 RNA polymerase to obtain a corresponding apoB RNA template, then DNase was added to digest the DNA template, and finally heated for 2min at 80°C for inactivation.

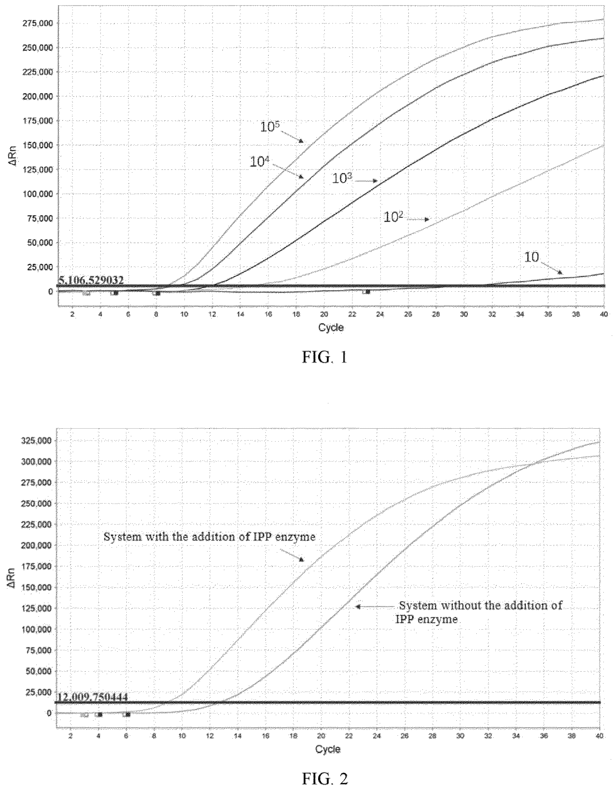

The test results after the addition of MMLV enzyme and RNasin were shown in Fig. 1. The numbers indicated by the arrows indicated that the test groups added with RNA templates for reaction at different concentrations. It could be seen that the intensity of the fluorescence amplification curve presented a gradient decline with the decrease of the concentrations (10⁵, 10⁴, 10³, 10², 10 copies/µL) of RNA templates for reaction, indicating that the RT-qRPA system could be used for the amplification of RNA templates, and the amplification efficiency would be increased with the increase of template concentration. The detection sensitivity could reach 10 copies/µL.

### Comparative example 1

The inventors have found that the addition of IPP enzyme in the reaction system can significantly improve the efficiency of the RPA reaction and thus improve the detection sensitivity.

Referring to the method in Example 1, the test results of the reaction system with and without adding IPP enzyme are shown in Fig. 2, and it can be clearly seen that the CT value of the fluorescence amplification curve of the RT-qRPA system after adding 1U/µL IPP enzyme is significantly higher than that of the RT-QRPA system, indicating that IPP enzyme can significantly improve the efficiency of RPA reaction.

The results of sensitivity test showed that the sensitivity of RT-RPA reaction system without adding IPP enzyme was only 10² copies/µL.

All references cited herein are incorporated by reference in this application as if each reference is individually incorporated by reference. Furthermore, it is to be understood that various changes or modifications may occur to those skilled in the art after reading the above teachings of the present invention, and that such equivalents fall within the scope of the appended claims.

## Claims

1. A reverse-transcription amplification reagent combination based on recombinase polymerase amplification technology, wherein the reagent combination comprises a recombinase, a single-chain binding protein, a DNA polymerase, a reverse transcriptase, and a ribonuclease inhibitor protein.

2. The reagent combination of claim 1, wherein the reagent combination further comprises an inorganic pyrophosphatase.

3. The reagent combination of claim 1, wherein the reagent combination further comprises a loading protein; preferably, the loading protein is loading protein UvsY.

4. The reagent combination of claim 1, wherein the reagent combination further comprises an exonuclease; preferably, the exonuclease is Exonuclease III (EXO).

5. The reagent combination of claim 1, wherein the reagent combination further comprises Creatine Kinase (CK).

6. The reagent combination of claim 1, wherein the recombinase is recombinase UvsX.

7. The reagent combination of claim 1, wherein the single-chain binding protein is single-chain binding protein gp32.

8. The reagent combination of claim 1, wherein the DNA polymerase is Bsu DNA polymerase.

9. A kit comprising the reagent combination of claim 1.

10. A method for reverse-transcription amplification of RNA in a sample based on recombinase polymerase amplification technology, the method comprising the steps of:
(1) preparing an RT-RPA amplification system
The RT-RPA amplification system comprises a reagent combination according to the first aspect of the invention, an RNA template, and a primer for specifically amplifying a reverse transcription product of the RNA template;
(2) amplification reaction
The amplification reaction is carried out under an isothermal condition.
